# EUROPEAN PATENT SPECIFICATION

(11) **EP 3 163 142 B1**
(45) Date of publication and mention of the grant of the patent: **26.09.2018**
(21) Application number: 16195723.8
(22) Date of filing: 26.10.2016
(51) Int. Cl.: F16L 37/14, A61M 16/20

(54) **DEVICE FOR CONTROLLED SUPPLY OF A MEDICAL GAS, WITH REPLACEABLE OUTLET UNION, AND METHOD FOR THE MANUFACTURE OF SAID DEVICE**
VORRICHTUNG ZUR GESTEUERTEN ZUFUHR EINES MEDIZINISCHEN GASES, MIT AUSWECHSELBAREM AUSLASSZUSAMMENSCHLUSS, UND VERFAHREN ZUR HERSTELLUNG DER BESAGTEN VORRICHTUNG
DISPOSITIF D'ALIMENTATION CONTRÔLÉE DE GAZ MÉDICAL, AVEC UN RACCORD UNION DE SORTIE REMPLAÇABLE, ET PROCÉDÉ DE FABRICATION DE CE DISPOSITIF

(30) Priority: 29.10.2015 IT UB20156493 U
(43) Date of publication of application: 03.05.2017
(73) Proprietor: Flow Meter S.p.a., 24040 Levate (Bergamo) (IT)
(72) Inventor: PARATICO, Roberto, 24040 Levate (BG) (IT)
(74) Representative: Botti, Mario

(56) References cited:
- EP-A1- 2 732 840
- FR-A1- 2 793 867
- US-A1- 2003 038 479
- US-A1- 2003 197 369
- US-A1- 2009 079 188

## Description

### Field of application

The present invention relates to a device for the controlled supply of a medical gas, in particular of the type designed to regulate and/or measure a gas flow from a supply network or from a pressurized vessel and supply one more user appliances.

In particular, the present invention relates to an instantaneous variable-area flowrate measuring device - otherwise called flowmeter - suitable for metering medical gases.

### Prior art

The regulation and/or measurement of the flowrate of a generic fluid flowing in circuits or pipes is necessary in various applications belonging to different technical sectors.

In particular, in the medical sector regulators and measuring devices of various kinds are used to meter the supply of pressurized gas intended for therapeutic applications, such as oxygen therapy.

These devices may be applied to the outlet of a supply network or, if equipped with a suitable high-pressure reducer which is integrated or connected in series, they may be applied to a pressurized vessel containing medical gas.

They therefore comprise a flow inlet, which is connected to the medical gas source, and a flow path which leads to one or more outlets.

Depending on the type of device, the flow path may be provided with one or more pressure reduction stages, an optional pressure gauge designed to read the pressure upstream of the device, a measuring element for reading the flowrate downstream of the device, and a flowrate regulator of the type with calibrated orifices or variable area (needle valve).

A threaded outlet union which allows connection to the user appliance is also provided at the flow path outlet.

Moreover, in the medical sector, except for some specific gases and in any case for limited applications, a single set of regulations defining unique globally recognized standards for threaded connections intended for the connection of appliances or accessories does not exist. There exists instead a series of national standards, which are not regulated or in some cases relate to technical regulations for applications other than the medical sector (even though typically used and recognized by the markets) which propose valid solutions for one or more countries.

In order to meet the demands of the international market, the producer must therefore provide a series of models which differ solely in terms of the type of outlet union, resulting in a substantial increase in the production and warehouse costs.

Moreover, the limited flexibility of use of the device constitutes a drawback also for the end users. The latter may in fact connect only certain types of appliances to the outlet of their device and must use specific reducers or adapters if they wish to connect an appliance with a non-compatible union to the same supply device.

In order to overcome this drawback, the Applicant has devised devices equipped with an outlet union which may be reconfigured, in particular having two opposite threaded connections which may be alternately used in an operating configuration. As disclosed in the corresponding patent EP 2 732 840 in the name of the same Applicant, a double-face threaded union is locked in a specific operating configuration by means of a shaped pin which is introduced into a transverse slit in the device and embraces a grooved connecting edge of the union.

The aforementioned devices, while satisfying in an excellent manner the need for operational flexibility discussed above, nevertheless have a production cost which is substantially higher than that of the other devices of the prior art.

The increased production costs arise in particular from the associated structural complexity of the grooved pin, which must be made of thermoplastic material using an injection-moulding process. It is therefore necessary to provide a special mould and an associated processing station, with a consequent substantial increase in the costs borne by the producer.

Other connection devices of an outlet union element and a main body involving a U-shaped locking pin are known from US 2003/038479A1, US 2003/0197369 A1 and FR 2793867 A1.
The technical problem forming the basis of the present invention is therefore that of devising a supply device which allows easy replacement of the outlet union, without involving however high manufacturing costs, and which moreover is easy-to-use for sanitary staff, is reliable and has a high mechanical strength.

### Summary of the invention

The aforementioned technical problem is solved by a device for controlled supply of a medical gas according to claim 1.

A person skilled in the art will easily understand how with the new configuration of the entire fastening mechanism it is possible to achieve significant savings in terms of production costs, without thereby negatively affecting the quality and strength of the device.

In particular, if the prior mechanism uses a form-fitting system where the entire cross-section of the pin or clip is introduced inside a transverse slit so as to embrace the outlet union, in the new system only two rigid prongs are introduced into a corresponding number of lateral guide holes, while the intermediate portion of the union performs the simple function of a grip for the user.

The locking pin may thus be made from an ordinary, small-diameter, metal bar by means of simple and low-cost cold machining operations: the rod needs only to be cut and bent to size at two points so as to define the prongs.

It should be noted that the use of a metal in order to produce the pin guarantees excellent strength and ductility properties, helping ensure the solidity of the fastening mechanism and facilitating the plastic deformation operations needed to produce the part.

The metal specifically used may be any metal with characteristics suitable for the purpose, for example a special stainless steel for springs.

Moreover machining of the casing, which is also preferably metallic, is significantly facilitated by the aforementioned configuration, two drilling operations being sufficient to define the transverse aperture for inserting the locking pin.

Furthermore, the lateral opening may also have more complex forms which may in any case be realized on a metal or plastic surface.

The lateral opening comprises a slit which connects the holes with each other and with an external surface of the main body, said slit concealingly housing said bridge-piece. Essentially, the slit is therefore a linear aperture with a thickness and length the same as the thickness and length of the bridge-piece, which is therefore inserted inside it so as to restore the continuity of the external surface of the main body. In the case of a rounded main body, the bridge-piece may be where necessary curved so as to match said rounded form.

The aforementioned slit, in addition to housing the bridge-piece, has the additional function of keeping the locking pin in its correct position, preventing rotational movements of the prongs inside the holes which receive them.

It should be noted that the main body may optionally comprise a metal structural core and plastic decorative lining. In this case, it is possible to consider forming the two holes in the metal structural core and the slit for housing the bridge-piece in the overlying plastic lining.

The lateral opening comprises a nail insertion recess facing said slit and designed to allow the introduction of a tool between a bottom surface of the slit and the bridge-piece of said locking pin, in order to facilitate removal of the locking pin from the main body during replacement of the outlet union.

The nail insertion recess has the form of an aperture transverse with respect to the slit, so as to allow linear access for the tip of the tool below the bridge-piece of the locking pin, namely between the latter and the bottom of the slit.

Preferably said cavity and said engaging shank have respective mating profiles which are counter-shaped and not axial symmetrical so that the engaging shank engages in a rotatably fixed manner inside said cavity. For example, the mating profile of the engaging shank may be polygonal or eccentric, mating with a corresponding polygonal or eccentric profile of the cavity receiving it.

The groove is preferably formed inside a cylindrical portion of the engaging shank arranged upstream with respect to said mating profile. The device may further comprise a sealing gasket pressed between a circumferential bearing surface formed at the end of said engaging shank and a bottom surface of the cavity.

It should be noted that the parts have dimensions such that, when the locking pin locks the outlet union in position, the sealing gasket is compressed between the bearing surface and the bottom of the cavity.

Preferably, the bearing surface is formed above a peripheral circumferential shoulder and is internally delimited by a tubular edge which ensures the continuity of the flow supply path of the device.

It should be noted that the device may advantageously comprise a plurality of outlet unions, the threaded heads of which have different configurations or comply with different standards, namely have different diameter sizes and/or thread parameters or in some cases also specific fastening systems. The outlet unions may be associated alternately, depending on the requirements which may arise, with said main body.

As already previously mentioned, the main body may be at least partially metallic, with two holes which cross transversely the metal portion of said main body.

The aforementioned technical problem is also solved by a method for manufacturing a device having the characteristic features described above.

Said method comprises, in addition to the production and assembly steps already used for the manufacture of similar devices, a specific step for manufacture of the locking pin performed by means of plastic deformation with bending of a metal rod at two points.

The term "metal rod" must not be understood here in a limiting sense and indicates a wire or bar with a more or less uniform diameter made of any metallic material.

The plastic deformation step may be preceded by a step for cutting to size and chamfering the ends of the metal rod.

The plastic deformation step comprises preferably two 90° bends in the metal rod which define a U-shaped mirror-symmetrical structure.

The method of manufacturing the device also comprises steps for forming the cavity and the lateral opening which are performed by means of milling and/or boring operations.

In particular, the cavity, as well as the external slit of the lateral opening, may be obtained by means of a milling operation. The two holes which connect the slit with the cavity are obtained by means of boring following milling of the slit.

Further characteristic features and advantages of the device for supplying medical gas according to the invention will emerge from the description, provided hereinbelow, of a non-limiting example of embodiment thereof with reference to the accompanying drawings.

### Brief description of the drawings

Figure 1 shows a perspective view of a variable-area flowmeter according to the present invention;
Figure 2 shows the flowmeter according to Figure 1 with outlet union disassembled;
Figures 3a-3e show perspective views of the successive operations for disassembly of an outlet union of the flowmeter according to Figure 1;
Figures 4a-4d show perspective views, partially sectioned along a centre plane, of the successive operations for disassembly of an outlet union of the flowmeter according to Figure 1.

### Detailed description

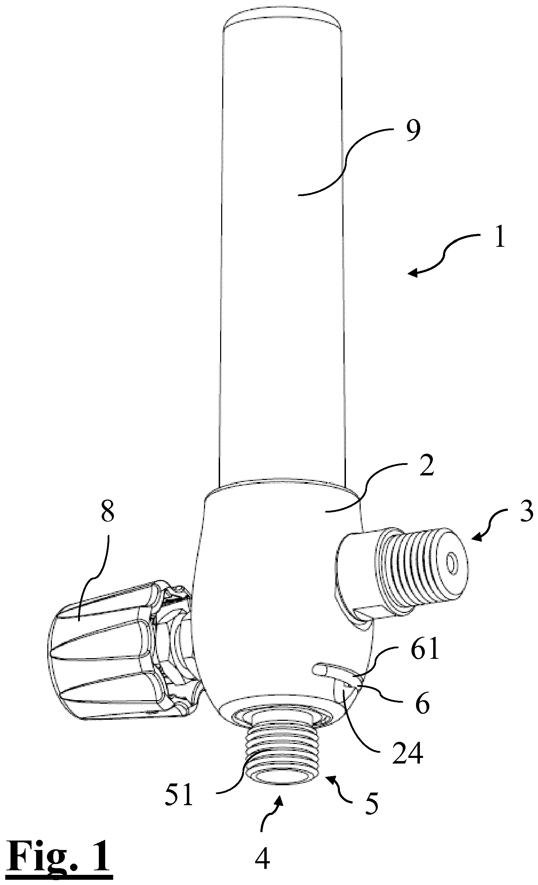

With reference to the attached figures 1 denotes generally a preferred embodiment of the device for supplying medical gas according to the present invention.

In this preferred embodiment, the device 1 consists of a flowmeter with needle valve, namely a device suitable for metering and measuring instantaneously the flowrate of a medical gas. It should be noted in any case that the device according to the present invention may be a supply device of another type, such as a pressure reducer or a flowmeter with calibrated orifices.

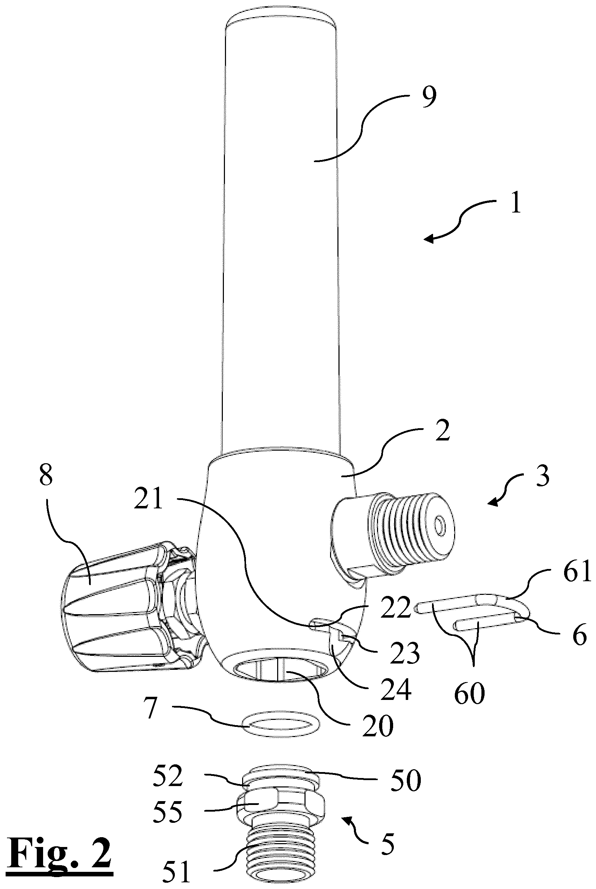

The flowmeter 1 is shown in Figures 1 and 2 in a normal operating configuration; in the continuation of the present description, the relative and absolute positions and the orientations of the various elements which form the unit - defined by means of terms such as "upper" and "lower", "above" and "below", "horizontal" and "vertical" or other equivalent terms - must always be interpreted with reference to this configuration.

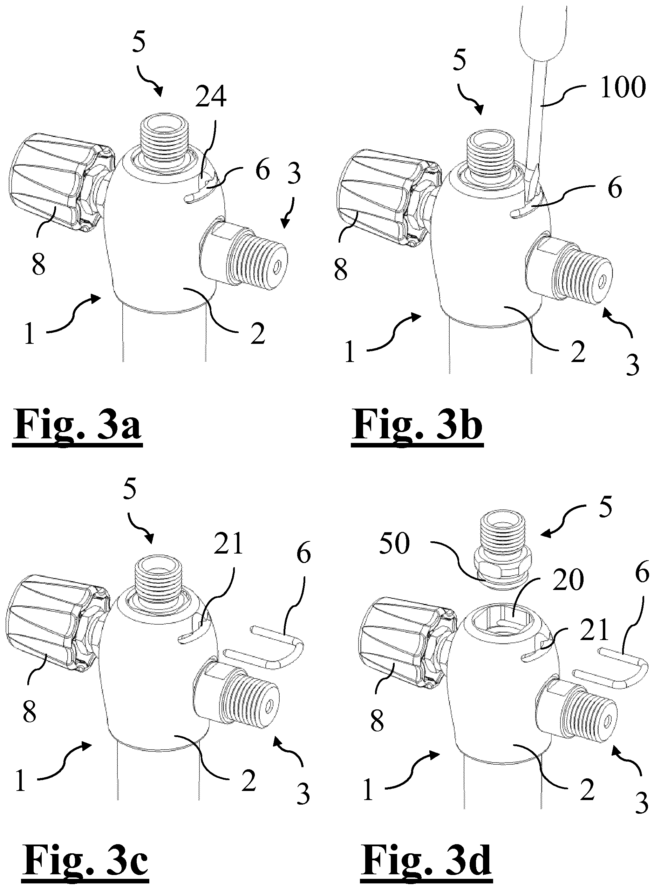

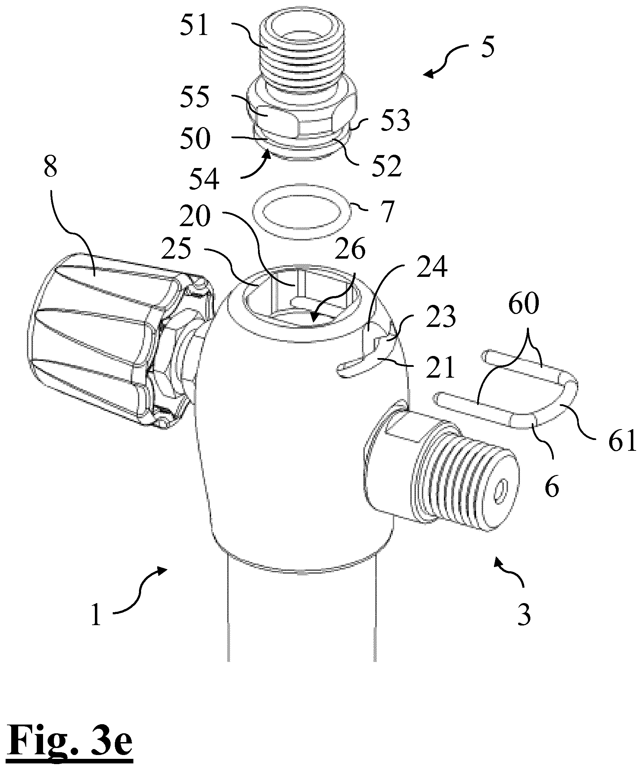

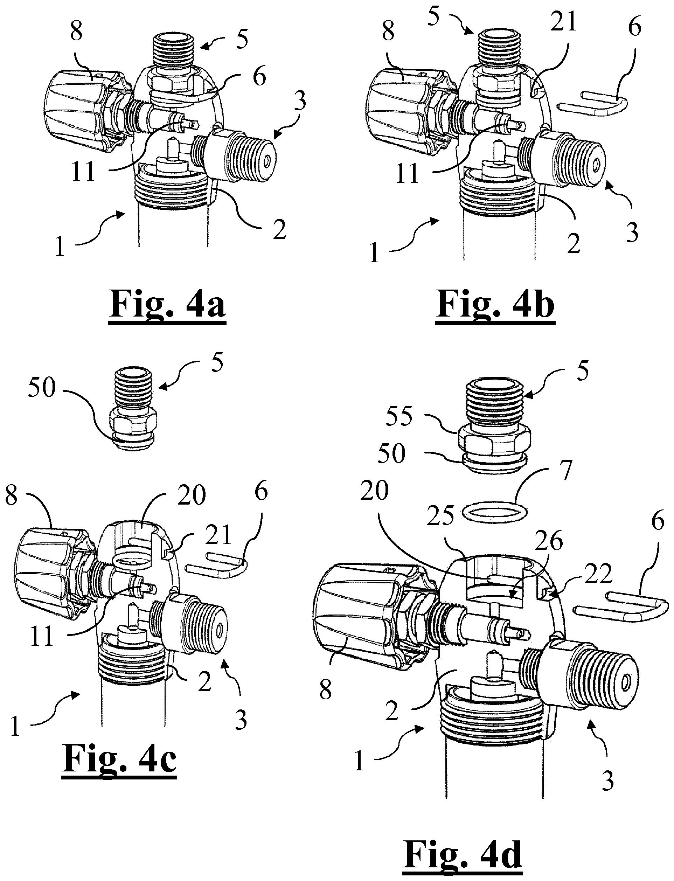

Figures 3a-3e, 4a-4d show instead the flowmeter in an overturned configuration.

The flowmeter 1 comprises a main body 2, with a supporting and containing function, inside which at least one flow supply path is defined, said flow path extending between an inlet 3, defined by a threaded connector (or specific quick-coupling gas connection) of the known type and an outlet 4 defined by a removable outlet union 5 described in detail below.

The main body 2 has the form of a curved drum or cylinder, extending along a substantially vertical direction which will be identified in the continuation of the present invention as longitudinal direction of the device. On the other hand, any reference to a direction or orientation transverse to the device indicates a direction perpendicular to the aforementioned longitudinal direction.

The main body 2 is preferably made as one piece from metallic material, for example anodized or chrome-plated aluminium; according to a (non-preferred) variant of the invention an outer lining made of plastic, for example polycarbonate or ABS, may be provided.

The threaded connector which defines the inlet 3 extends transversely with respect to the main body 2; a regulating knob 8 connected to a needle valve 11 which controls the gas flow along the flow supply path is provided opposite said inlet.

On the other hand, along the longitudinal axis, a frustoconical pipe 9 with an indicator element for measuring the flowrate is provided on one side and the removable outlet union 5 is provided on the other side.

The outlet union 5 has an internally continuous tubular structure, while externally it is divided into a upper engaging shank 50 which is fixed inside a cavity 20 of the main body 2 and an opposite externally threaded head 51 designed to be screwed in a known manner together with appliances and/or pipes arranged downstream of the flowmeter.

It should be noted that the threaded head 51 complies with specific dimensions and configurations, namely has predefined lengths, forms, diameters and/or thread parameters. The device may have alternative outlet unions 5 where the engaging shank remains identical, but the threaded head meets a different standard. By replacing the outlet union 5 it is thus possible to adapt to a specific local or international standard and/or a specific device.

The engaging shank 50 comprises, directly above the threaded head 51, a nut or a mating profile 55 with a substantially square shape suitable for ensuring torsional resistance and designed to be introduced with a form fit inside a corresponding mating profile 25 of the cavity 20.

An outer cylindrical portion 53 extends above the mating profile 55 and is interrupted only by an intermediate circumferential shoulder. A circumferential groove 52 is defined between the shoulder and the underlying mating profile 55; a bearing surface 54 for positioning a toroidal sealing gasket 7 is instead defined above the shoulder.

When the outlet union 5 is mounted in position, the sealing gasket 7 is pressed between the underlying bearing surface 54 and the bottom 26 of the cavity 20 situated above.

The device 1 also comprises a locking pin 6 which allows the outlet union 5 to be locked in position inside the cavity 20.

The locking pin 6 consists of a rod made of metallic material and bent in the form of a "U" and has in particular two straight prongs 6 which are connected together by an intermediate portion or bridge-piece 61.

The main body 2 has, on the side where the inlet 3 is located, a transverse lateral opening 21 inside which said locking pin 6 is engaged.

The lateral opening 21 comprises a slit 23, which extends through only the outermost surface of the main body 2, stopping at a bottom surface, at a distance from an inner surface of the cavity 20. The lateral opening 21 extends along a circumferential segment of the main body 2.

A corresponding number of holes 22 which connect the slit 23 with the inside of the cavity 20 are provided at the two opposite ends of the bottom of the slit 23.

The locking pin 6 is introduced by inserting the two prongs 60 inside the respective holes 22, while the bridge-piece 61 is concealingly inserted inside the slit 23. The free end of the prongs 60 thus inserted laterally engages inside the grooves 52 on the two sides of the outlet union 5, locking it in position.

In a lower position with respect to the transverse slit 23, the external surface of the main body 2 has formed therein a nail insertion recess 24 which connects the said slit to the sloping bottom external surface.

Via the nail insertion recess 24 it is possible to introduce a tool - for example the tip of a screwdriver 100, as shown in Figure 3b - in order to exert a lever action between the bottom of the slit 23 and the locking pin 6, thus facilitating extraction of the latter.

Reference is made briefly below to the method for manufacturing the aforementioned flowmeter, which may advantageously comprise a simple step of cutting to size and imparting a double 90° bend to the metal rod in order to form the locking pin 6.

The cavity 20 and slit 23, as well as the nail insertion recess 24, may be formed by means of milling; the two holes 22 are then subsequently formed by boring the bottom of the previously milled slit 23.

Obviously a person skilled in the art, in order to satisfy any specific requirements which might arise, may make numerous modifications and variations to the flowmeter described above, all of which are contained moreover within the scope of protection of the invention, as defined by the following claims.

## Claims

1. Device (1) for controlled supply of a medical gas, comprising a main body (2) inside which at least one flow supply path extending between an inlet (3) and an outlet (4) is defined; an outlet union (5) which allows connection of the outlet (4) of said flow supply path to a path and/or an appliance downstream of said outlet; said outlet union (5) being associated in a separable manner with the main body (2); said outlet union (5) comprising an engaging shank (50) inserted inside a cavity (20) of the main body (2) and an opposite threaded head (51) external with respect to the main body (2); said device further comprising a locking pin (6); said outlet union (5) being removably fixed inside said cavity (20) by means of the locking pin (6) introduced transversely inside a lateral opening (21) interfering with said cavity (20); **characterized in that** said locking pin (6) is formed by a metal rod bent in the form of a "U" so as to define two prongs (60) connected by a bridge-piece (61), the two prongs (60) being introduced inside a corresponding number of holes (22) of the lateral opening (21) and laterally engaging inside a groove (52) formed in the engaging shank (50) so as to lock in position said outlet union (5), the bridge-piece (61) being accessible from the outside of the main body (2) so as to allow a user to extract the locking pin (6) and thus remove the outlet union (5); wherein said lateral opening (21) further comprises a slit (23) which connects said holes with each other and with an outer surface of the main body (2), said slit (23) concealingly housing said bridge-piece (61); wherein said lateral opening (21) further comprises a nail insertion recess (24) facing said slit (23) and designed to allow the introduction of a tool (100) between the bottom of the slit (23) and the bridge-piece (61) of said locking pin (6).

2. Device (1) according to one of the preceding claims, wherein said cavity (20) and said engaging shank (50) have respective mating profiles (25, 55) which are counter-shaped and not axial symmetrical so that the engaging shank (50) engages in a rotatably fixed manner inside said cavity (20).

3. Device (1) according to claim 2, wherein said groove (52) is formed inside a cylindrical portion (53) of the engaging shank (50) arranged upstream of said mating profile (55).

4. Device (1) according to claim 3, further comprising a sealing gasket (7) pressed between a circumferential bearing surface (54) formed at the end of said engaging shank (50) and a bottom surface (26) of the cavity (20).

5. Device (1) according to one of the preceding claims, comprising a plurality of outlet unions (5), the threaded heads (51) of which comply with different standards, i.e. have different sizes of length, shape, diameter and/or thread parameters, the outlet unions (5) being able to be associated alternately with said main body (2).

6. Device (1) according to one of the preceding claims, wherein said main body (2) is at least partially metallic, with two holes (22) which pass through transversely the metal portion of said main body (2).

7. Method for manufacturing a device (1) according to one of the preceding claims, wherein said locking pin (6) is obtained by means of plastic deformation by bending a metal rod at two points.

8. Method according to claim 7, wherein said cavity (20) and said lateral opening (21) are obtained by means of milling and/or boring operations.

## Patentansprüche

1. Vorrichtung (1) für die gesteuerte Zufuhr eines medizinischen Gases, mit einem Hauptkörper (2), in dem mindestens ein sich zwischen einem Einlass (3) und einem Auslass (4) sich erstreckender Strömungszufuhrweg gebildet ist; einer Auslasskupplung (5), die eine Verbindung des Auslasses (4) des Strömungszufuhrweges mit einem Weg und/oder Gerät stromabwärts von dem Auslass erlaubt; wobei die Auslasskupplung (5) auf eine trennbare Art und Weise mit dem Hauptkörper (2) verbunden ist; wobei die Auslasskupplung (5) einen Eingriffsschaft (50), der in einen Hohlraum (20) des Hauptkörpers (2) eingeführt ist, und einen entgegengesetzten Gewindekopf (51) außerhalb des Hauptkörpers (2) aufweist; wobei die Vorrichtung außerdem einen Verriegelungsstift (6) aufweist; wobei die Auslasskupplung (5) lösbar in dem Hohlraum (20) mittels des Verriegelungsstiftes (6), der quer in eine seitliche Öffnung (21) eingesetzt ist, die sich mit dem Hohlraum (20) kreuzt, befestigt ist; **dadurch gekennzeichnet, dass** der Verriegelungsstift (6) aus einem Metallstab geformt ist, der in Form eines "U" gebogen ist, um zwei Zinken (60) zu bilden, die durch ein Brückenstück (61) miteinander verbunden sind, wobei die beiden Zinken (60) in eine entsprechende Anzahl von Löchern (22) der seitlichen Öffnung (21) eingeführt sind und in eine in dem Eingriffsschaft (50) geformte Rille (52) seitlich eingreifen, um die Auslasskupplung (5) in Position zu halten, wobei das Brückenstück (61) von der Außenseite des Hauptkörpers (2) her zugänglich ist, um einem Benutzer zu gestatten, den Verriegelungsstift (6) herauszuziehen und somit die Auslasskupplung (5) zu entfernen; wobei die seitliche Öffnung (21) außerdem einen Schlitz (23) aufweist, der die Löcher miteinander und mit einer Außenfläche des Hauptkörpers (2) verbindet, wobei der Schlitz (23) das Brückenstück (61) verdeckt aufnimmt; wobei die seitliche Öffnung (21) außerdem eine Nageleinführausnehmung (24) aufweist, die auf den Schlitz (23) weist und gestaltet ist, um die Einführung eines Werkzeuges (100) zwischen dem Boden des Schlitzes (23) und dem Brückenstück (61) des Verriegelungsstiftes (6) zu gestatten.

2. Vorrichtung (1) nach einem der vorhergehenden Ansprüche, bei der der Hohlraum (20) und der Eingriffsschaft (50) jeweils zusammenpassende Profile (25, 55) haben, die zueinander entgegengesetzt geformt und nicht axial symmetrisch sind, so dass der Eingriffsschaft (50) auf eine drehfeste Art und Weise in den Hohlraum (20) eingreift.

3. Vorrichtung (1) nach Anspruch 2, bei der die Rille (52) in einem zylindrischen Teil (53) des Eingriffsschaftes (50) geformt ist, der stromaufwärts von dem zusammenpassenden Profil (55) angeordnet ist.

4. Vorrichtung (1) nach Anspruch 3, ferner mit einem Dichtungsring (7), der zwischen einer umlaufenden Lagerfläche (54), die an dem Ende des Eingriffsschaftes (50) gebildet ist, und einer Bodenfläche (26) des Hohlraumes (20) zusammengepresst ist.

5. Vorrichtung (1) nach einem der vorhergehenden Ansprüche, mit einer Vielzahl von Auslasskupplungen (5), von denen die Gewindeköpfe (51) verschiedenen Normen entsprechen, d.h. verschiedene Größen in der Länge, in der Form, im Durchmesser und/oder in den Gewindeparametern haben, wobei die Auslasskupplungen (5) wechselweise mit dem Hauptkörper (2) verbunden werden können.

6. Vorrichtung (1) nach einem der vorhergehenden Ansprüche, bei der der Hauptkörper (2) mindestens zum Teil metallisch ist, mit zwei Löchern (22), die quer durch den Metallteil des Hauptkörpers (2) gehen.

7. Verfahren zum Herstellen einer Vorrichtung (1) nach einem der vorhergehenden Ansprüche, wobei der Verriegelungsstift (6) durch plastische Verformung durch Biegen eines Metallstabes an zwei Punkten erhalten wird.

8. Verfahren nach Anspruch 7, bei dem der Hohlraum (20) und die seitliche Öffnung (21) durch Fräs- und/oder Bohrvorgänge erhalten werden.

## Revendications

1. Dispositif (1) pour l'alimentation contrôlée d'un gaz médical, comprenant un corps principal (2) à l'intérieur duquel est défini au moins un trajet d'alimentation s'étendant entre une entrée (3) et une sortie (4) ; un raccord de sortie (5) qui permet la connexion de la sortie (4) du trajet d'alimentation à un trajet et/ou un appareil placé en aval de ladite sortie ; le raccord de sortie (5) étant relié de manière séparable au corps principal (2) ; le raccord de sortie (5) comprenant une queue d'engagement (50) insérée à l'intérieur d'une cavité (20) du corps principal (2) et une tête filetée opposée (51) extérieure par rapport au corps principal (2) ; le dispositif comprenant en outre une goupille de verrouillage (6) ; le raccord de sortie (5) étant fixé de manière amovible à l'intérieur de la cavité (20) au moyen de la goupille de verrouillage (6) introduite transversalement à l'intérieur d'une ouverture latérale (21) qui interfère avec la cavité (20) ; **caractérisé en ce que** la goupille de verrouillage (6) est formée par une tige métallique pliée en forme de "U" de manière à définir deux branches (60) reliées par un élément en pont (61), les deux branches (60) étant introduites à l'intérieur d'un nombre correspondant de trous (22) de l'ouverture latérale (21) et s'engageant latéralement dans une rainure (52) formée dans la queue d'engagement (50) de sorte à verrouiller en position le raccord de sortie (5), l'élément en pont (61) étant accessible depuis l'extérieur du corps principal (2) de sorte à permettre à un utilisateur d'extraire la goupille de verrouillage (6) et ainsi d'enlever le raccord de sortie (5) ; l'ouverture latérale (21) comprenant en outre une fente (23) qui relie les trous entre eux et avec une surface extérieure du corps principal (2), ladite fente (23) logeant de manière dissimulée l'élément en pont (61) ; l'ouverture latérale (21) comprenant en outre un évidement d'insertion d'ongle (24) faisant face à la fente (23) et conçu pour permettre l'introduction d'un outil (100) entre le fond de la fente (23) et l'élément en pont (61) de la goupille de verrouillage (6).

2. Dispositif (1) selon l'une des revendications précédentes, dans lequel la cavité (20) et la queue d'engagement (50) présentent des profils conjugués respectifs (25, 55) qui sont de forme opposée et non symétriques axialement de sorte que la queue d'engagement (50) s'engage de manière fixe en rotation à l'intérieur de la cavité (20).

3. Dispositif (1) selon la revendication 2, dans lequel la rainure (52) est formée à l'intérieur d'une partie cylindrique (53) de la queue d'engagement (50) disposée en amont du profil conjugué (55).

4. Dispositif (1) selon la revendication 3, comprenant en outre un joint d'étanchéité (7) pressé entre une surface d'appui circonférentielle (54) formée à l'extrémité de la queue d'engagement (50) et une surface de fond (26) de la cavité (20).

5. Dispositif (1) selon l'une des revendications précédentes, comprenant une pluralité de raccords de sortie (5) dont les têtes filetées (51) répondent à des normes différentes, c'est-à-dire qu'elles ont des dimensions de longueur, des formes, des diamètres et/ou des paramètres de filetage différents, les raccords de sortie (5) pouvant être associés alternativement au corps principal (2).

6. Dispositif (1) selon l'une des revendications précédentes, dans lequel le corps principal (2) est au moins partiellement métallique, avec deux trous (22) qui traversent transversalement la partie métallique dudit corps principal (2).

7. Procédé de fabrication d'un dispositif (1) selon l'une des revendications précédentes, dans lequel la goupille de verrouillage (6) est obtenue par le biais d'une déformation plastique par pliage en deux points d'une tige métallique.

8. Procédé selon la revendication 7, dans lequel ladite cavité (20) et l'ouverture latérale (21) sont obtenues au moyen d'opérations de fraisage et/ou de perçage.
